Europäisches Patentamt

European Patent Office

Office européen des brevets

(11) Publication number: **0 314 274 B1**

## EUROPEAN PATENT SPECIFICATION

(45) Date of publication of patent specification: **06.04.94**

(51) Int. Cl.⁵: **C12N 15/70**, C12N 15/67, C12N 1/20, //(C12N1/20, C12R1:19)

(21) Application number: **88307215.9**

(22) Date of filing: **04.08.88**

(54) **Improved promoter, vector having the improved promoter, plasmid having the vector, and E.coli transformed with the plasmid.**

(30) Priority: **05.08.87 JP 194524/87**

(43) Date of publication of application:
**03.05.89 Bulletin 89/18**

(45) Publication of the grant of the patent:
**06.04.94 Bulletin 94/14**

(84) Designated Contracting States:
**BE CH DE FR GB IT LI NL SE**

(56) References cited:
**EP-A- 0 180 964**
**FR-A- 2 598 430**

**Nucleic Acids Research, vol. 11, no. 8, pp. 2237-2255, 1983, IRL Press Ltd., Oxford, GB; D.K. Hawley et al.**

**The EMBO Journal, vol. 6, no. 10, pp. 3139-3144, 1987, IRL Press Ltd., Oxford; GB; M. Brunner et al.**

(73) Proprietor: **UBE INDUSTRIES, LTD.**
**12-32, Nishihonmachi 1-chome**
**Ube-shi, Yamaguchi-ken 755(JP)**

(72) Inventor: **Tanaka, Hiroshi**
**Ube Res. Lab. of Ube Ind. Ltd.**
**1978-5,Oaza Kogushi**
**Ube-shi Yamaguchi-ken(JP)**
Inventor: **Anpeiji, Shigeharu**
**Ube Res. Lab. of Ube Ind. Ltd.**
**1978-5,Oaza Kogushi**
**Ube-shi Yamaguchi-ken(JP)**
Inventor: **Matsuda, Akihisa**
**Ube Res. Lab. of Ube Ind. Ltd.**
**1978-5,Oaza Kogushi**
**Ube-shi Yamaguchi-ken(JP)**

(74) Representative: **Myerscough, Philip Boyd et al**
**J.A.KEMP & CO.**
**14, South Square**
**Gray's Inn**
**London WC1R 5LX (GB)**

**Description**

This invention relates to an improved promoter of E. coli, a vector having the improved promoter, a plasmid having the vector, and E. coli transformed with the plasmid. More specifically, it relates to an improved promoter instructing excessive production of proteins, a vector having the improved promoter, a plasmid having the vector, and E. coli transformed with the plasmid.

In recent years, attempts have actively been made to obtain physiologically active substances useful to mankind not by separation and extraction from biological sources but by culturing animal cells and transformed microorganisms at low costs on an industrial scale.

The production of physiologically active substances using microorganisms can be performed on an industrial scale by utilizing the fermentation technology accumulated in the art up to date. Accordingly, industrial production of physiologically active substances depends upon whether useful transformed microorganisms can be produced by using the gene recombinant technology.

Any useful transformed microorganisms should efficiently produce large quantities of the desired physiologically active substances. For this purpose, the structural genes of the physiologically active substances should be transcribed and translated efficiently in the tranformed microorganisms. The resulting products should retain the native structure of the active substances.

Methods of efficiently transcribing structural genes include, for example, conversion of the genetic codes of the structural genes themselves into those which can be efficiently utilized by the transformed microorganisms, increasing of the affinity of RNA polymerase for the promoter, and amplification of the plasmid bearing the structural genes for increasing the product in the microorganisms.

On the other hand, to perform translation of the genes efficiently, there is, for example, a method which strengthen the binding of ribosome to a ribosome-binding site or a method by which sites capable of inhibiting translation are removed.

The gene technology on transcription and translation is important, and so far attempts have been actively made to enhance the efficiency in the step of expressing a structural genes.

A conventional method of expressing structural genes by gene technology to join natural promoters obtained by the degestion with suitable restriction endonucleases, for example a tryptophan promoter (trp promoter), a lactose promoter (lac promoter) or colicin E1 promoter (col E1 promoter) to the upstream end of the structural gene. The efficiency of expression obtained by using these promoters generally remain within the range of the efficiency of expression of structural genes to which conventional natural promoters are bonded, and are not greatly different.

A number of data have been accumulated on the DNA base sequences of E. coli promoters. These data show that a consensus base sequence represented by

$$5'-TTGACA-3'$$
$$AACTGT$$

exists in a region of 35 bases (to be referred to as the "-35 region") upstream of the transcription initiation point and a consensus base sequence represented by

$$5'-TATAAT-3'$$
$$ATATTA$$

exists in a region of 10 bases (to be referred to as the "-10 region") upstream of the transcription initiation point commonly in these E. coli promoters.

It has been found that the transcription efficiency of the structural genes is higher as the base sequence of the promoter is nearer to the above common base sequences and the number of bases G and C in the base sequence of a region between the "-35 region" and the "-10 region" (referred to as the "intermediate region") is smaller [Hawlei et al.: Nucleic Acid Res., 11 (8), 2237 (1983)].

A combined promoter (tac promoter) composed of the "-35 region" of trp promoter and the "-10 region" of lacUV-5 promoter is known as an improved promoter having the aforesaid base sequence obtained from a natural promoter.

The excessive production of a physiologically active substance, which is considered to damage the host is preferably carried out after the host is grown in a condition in which the production of the physiologically active substance is inhibited and then this inhibition is cancelled under suitable conditions.

2

Colicin E1 promoter (col E1 promoter) is one promoter which is suitable for performing the excessive production of a physiologically active substance by the above method.

Japanese Laid-Open Patent Publication No. 111690/1986 describes a method for producing human superoxide dismutase, a physiologically active substance, using col E1 promoter. An improved promoter obtained by artificially mutating the base sequence of col E1 promoter has not been known. It is therefore unknown how such an improved promoter, when it is actually obtained, will change the expression efficiency of a structural gene.

It is an object of this invention to provide a novel promoter by improving a promoter of E. coli.

Another object of this invention is to produce and provide an improved promoter which instructs the excessive production of proteins by artificially mutating the base sequence of a naturally occurring promoter.

Still another object of this invention is to provide an improved promoter having a much higher expression efficiency of protein production than a natural promoter such as col E1 promoter without losing the inherent specific regulation of the promoter which induces production by natural promoter, for example by irradiating ultraviolet light on it or treating it with mitomycin C.

A further object of this invention is to provide a vector having the improved promoter of the invention, a plasmid having the vector and an E. coli strain transformed with the plasmid.

Other objects of this invention along with its advantages will become apparent from the following description.

According to this invention, the above objects and advantages of the invention are achieved by an improved promoter of E. coli, in which

(a) the -35 region in the promoter shows the following base sequence

$$5'-TTGACAX-3'$$
$$AACTGTY$$

wherein the XY base pair represents a base pair of either TA, GC, AT or CG, and

the -10 region in the promoter shows the following base sequence

$$5'-TATAAT-3'$$
$$ATATTA$$

and,

(b) an intermediate region between the -35 region and the -10 region in the promoter consists of 15 to 17 base pairs and shows such a base sequence in which the proportion of base pairs CG and GC in the entire base pairs in this region is at least 60 %.

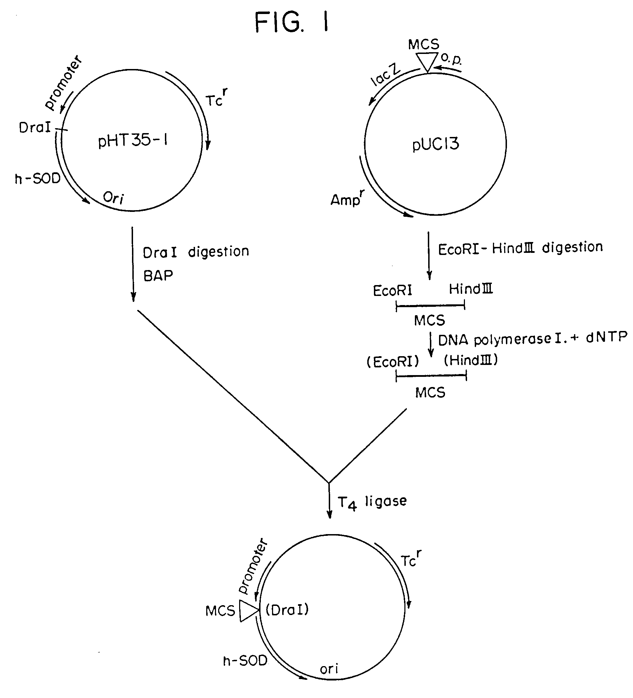

Figure 1 accompanying this application shows a summary of the process for constructing a high expression vector having a multiple cloning site (MCS) from plasmid vector pHT35-1 of the invention.

The improved promoter of the invention which instructs the excessive production of a physiologically active substance consists of the "-35 region", the "-10 region" and the "intermediate region".

The "-35 region" has the following base pair sequence.

$$5'-TTGACAX-3'$$
$$AACTGTY$$

The XY base pair in the above sequence represents a base pair of either TA, GC, AT or CG, preferably AT or GC.

The "-35 region" is composed of 7 base pairs from the -30 base pair (XY in the right end of the above sequence) to the -36 base pair (TA at the left end of the above sequence).

3

The "-10 region" has the following base sequence.

$$5'-TATAAT-3'$$
$$ATATTA$$

The "-10 region" is composed of six base pairs from the -8 base pair (TA at the right end in the above sequence) to the -13 base pair (TA at the left end of the above sequence).

The "intermediate region" is composed of 15 to 17 base pairs, preferably 16 base pairs. In the "intermediate region", the proportion of GC and CG base pairs in the entire base pairs is at least 60 %.

The promoter of this invention preferably has any one of the following base sequence as the base sequences of the "-35 region", the "intermediate region" and the "-10 region".

$$5'-TTGACATGGAAAATGCAGCGGCGTATAAT-3'$$
$$AACTGTACCTTTTACGTCGCCGCATATTA \quad , \quad and$$

$$5'-TTGACAGGGAAAATGCAGCGGCGTATAAT-3'$$
$$AACTGTCCCTTTTACGTCGCCGCATATTA \quad ,$$

The improved promoter can be prepared by properly combining DNA fragments (obtained by digestion with suitable restriction endonucleases) of natural promoters such as trp promoter, lac promoter, tac promoter, $\lambda P_L$ promoter and col E1 promoter. It can also be obtained by combining suitable chemically synthesized DNA fragments (which can be obtained, for example, by using an automatic DNA synthesizing device using the amidite method). It can of course be obtained by chemical synthesis alone.

The base sequence of the "intermediate region" is desirably caused to retain the function of expressing a structural gene linked downstream of the promoter by using ultraviolet light, mitomycin C, etc. which damage DNA.

In order to provide the promoter of this invention as a general-purpose promoter which can be utilized in the production of various physiologically active substances such as human superoxide dismutase, it is preferred to link a DNA fragment having cleavage sites with a plurality of restriction endonucleases to the downstream end of the "-10 region". Such an DNA fragment preferably has sites of cleavage with as many restriction endonucleases as possible, such as SstI, SmaI, XmaI, BamHI, XbaI, SalI, AccI and HincII. For example, a DNA fragment of pUC13 (a product of Pharmacia Co.) or chemically synthesized DNA fragments can be used.

Production of a physiologically active substance is carried out by linking a DNA fragment containing this structural gene to the downstream end of the improved promoter of the invention, inserting the DNA fragment into a plasmid having a large copy number or a plasmid whose copy number increases by an induction operation (such as pBR322 having high versatility), transforming a microorganism such as E. coli with the recombinant plasmid, selecting the transformed microorganism by colony hybridization or measuring the amount of the physiologically active substance, and cultivating the transformed microorganism.

In the following, the present invention will be described with regard to an example in which improved col E1 promoter has human Cu-Zn-superoxide dismutase (human Cu.Zn-SOD for short) as a structural gene downstream of the promoter, and pBR322 is used as a plasmid. The structural gene is a nucleotide sequence encoding the desired protein, and may be a structural gene isolated from nature, a structural gene chemically synthesized, or a structural gene obtained by a combination of the two methods. Where the use of conventional techniques is sufficient, no specific description is made.

The following Examples illustrate the present invention more specifically. It should be understood however that these examples do not limit the scope of the invention.

EXAMPLE 1

Chemical synthesis of single-stranded oligonucleotide

The following two synthetic oligonucleotides were used in this invention.

```
5'-GTCTTGACATGGAAAATGC-3'
5'-AGCGGCGTATAATTTATGCTG-3'
```

The above two oligonucleotides were synthesized by an automatic DNA synthesizer (Beckman System I DNA Synthesizer) in which the amidite method was used for synthesis.

EXAMPLE 2

Purification of synthetic oligonucleotides and phosphorylation of their end

After the synthesis of oligonucleotides in Example 1, the following steps were carried out to purify the oligonucleotides and phosphorylating the end.

1) To a silica gel carrier were added 200 microliters of thiophenol, 400 microliters of triethylamine and 400 microliters of dioxane, and with occasional shaking, the mixture was left to stand at room temperature for 1 hour.

2) After removing the solution, the carrier was washed with 1 ml of dioxane twice, 1 ml of methanol twice and 1 ml of ether once. Then, the carrier was dried with nitrogen gas.

3) One milliliter of cold aqueous ammonia was added to the carrier, and with occasional stirring the mixture was left to stand at room temperature for 3 hours.

4) The carrier was precipitated by centrifugation or standing, and the aqueous ammonia was recovered as the supernatant. The carrier was washed with freshly added aqueous ammonia (0.5 ml), and 1.5 ml in total of the aqueous ammonia as the supernatant was obtained.

5) The aqueous ammonia as the supernatant was transferred to a siliconized test tube, and the tube was sealed up by heat.

6) The sealed test tube was incubated for 12 hours or overnight at 50 to 55 °C.

7) The test tube was fully cooled in ice water, and then opened. The contents were devided into two 1.5 ml Eppendorf tubes and dried to a solid under vacuum.

8) The entire dried solid was dissolved in 300 microliters of sterilized water.

9) Butanol (1 ml) was added to extract the impurities. By repeating the operation twice, the synthetic oligonucleotide was obtained as a precipitate.

10) The synthetic oligonucleotide obtained as a precipitate was re-dissolved in 300 microliters of 0.3M sodium acetate solution and precipitated with ethanol.

11) The resulting precipitate was dissolved in 200 microliters of 7M urea to prepare a sample for separating acrylamide electrophoresis.

Composition of the gel

18 % Polyacrylamide (40:1.3 = acrylamide and methylenebisacrylamide)
7M urea
100 mM Tris-borate (pH 8.3)
2 mM EDTA
0.1 % (W/V) ammonium persulfate

12) The gel prepared with the above composition was pre-run for 20 minutes. A synthetic oligonucleotide solution was applied to the gel, and electrophoresis was carried out (C.V. 20 V/cm).

13) The electrophoresis was stopped when the distance between bands of bromophenol blue and xylene cyanol used as markers was broadened to about 5 cm.

14) The gel was placed on a TLC plate containing a fluorescent indicator and ultraviolet light of shortwave lengths was applied to it to visualize the bands, and the desired region was cut out.

15) The acrylamide gel fragment was finely crushed through the narrow opening a syringe and suspended in 1 ml of 1.0mM EDTA and 0.2M triethyl ammonium bicarbonate (pH 8.0).

16) The suspension was left to stand overnight at 37 °C, and centrifuged to recover a supernatant fraction.

17) The supernatant was applied to a DE52 column (1.0-1.5 cm) equilibrated with 0.2M triethyl ammonium bicarbonate (TEAB) (pH 8.0). The column was washed with 3-4 ml of 0.2M TEAB, and then eluted with 1.5 ml of 2M TEAB. One milliliter of the eluate which first obtained was recovered.

18) To the recovered synthetic oligonucleotide solution was added 10 times its volume of sterilized water, and the mixture was lyophilized to give a purified synthetic oligonucleotide.

19) A suitable amount of the dried synthetic oligonucleotide was dissolved in water.

20) The terminii of the synthetic oligonucleotide were phosphorylated by a known method.

EXAMPLE 3

Preparation of a vector containing improved colicin E1 promoter

In this example, site-specific mutation was carried out using the plasmid method.

Known plasmid pUBE2 described in Japanese Laid-Open Patent Publication No. 111690/1986 was digested with PvuII, and the cleavage site was dephosphorylated with bacterial alkaline phosphatase (the resulting DNA fragment is referred to as the DNA fragment I). On the other hand, plasmid pUBE2 was digested with SspI and StuI. The resulting large DNA fragment (DNA fragment containing a tetracycline-resistance gene; designated as the DNA fragment II) was separated. A reaction solution of the following formulation was prepared by using the phosphorylated oligonucleotide obtained in Example 2.

Formulation of the reaction solution

| | |
|---|---|
| DNA fragment I | 0.3 microgram |
| DNA fragment II | 0.26 microgram |
| 5-fold concentrated poly merase ligase buffer | 12 microliters |
| Phosphorylated synthetic oligonucleotide (10 pmol/microliter) | 3.75 microliters |
| Water | X microliters |
| Total | 34.8 microliters |
| (5-fold concentrated polymerase ligase buffer) 500 mM NaCl 32.5 mM Tris-HCl (pH 7.5) 40 mM MgCl$_2$ 5 mM beta-mercaptoethanol | |

A 11.6 microliter portion of the above reaction solution was separately kept as a control without boiling. The remaining portion (23.2 microliters) was boiled for 3 minutes, and stepwise cooled to 30 °C over 30 minutes and then to 4 °C over 30 minutes. Then, the cooled solution was left to stand in ice for 10 minutes. A 11.6 microliter portion of the reaction solution so treated was electrophoresed on a 0.7 % agarose gel together with the non-treated reaction solution. Electrophoresis was carried out in E buffer [40 mM Tris-acetate (pH 8.0), 20 mM sodium acetate and 2 mM EDTA] at 6 V/cm for 2 to 3 hours.

After confirming the formation of a heteroduplex, it was reacted overnight with a reaction solution of the following formulation at 12.5 °C.

Formulation of the reaction solution

| Heat-treated reaction solution | 11.6 microliters |
|---|---|
| 10 mM ATP | 2 microliters |
| Klenow enzyme (5 U/microliter) | 0.4 microliter |
| T$_4$ DNA ligase (0.5 U/microliter) | 2 microliters |
| 2.5 mM dNTP | 4 microliters |
| Total | 20 microliters |
| (2.5 mM dNTP shows a solution containing dTTP, dCTP, dGTP and dATP each in a concentration of 2.5 mM.) | |

An E. coli strain was transformed with the plasmid vector prepared by the above method to obtain transformants containing the plasmid vector.

EXAMPLE 4

Screening of the transformants containing the mutated plasmid vector

Colony hybridization was used as a screening method.

1) The transformants obtained in Example 3 were inoculated in an agar medium, and grown at 37 °C until the diameter of the colonies became 0.5 to 1 mm.

2) An autoclaved nitrocellulose filter was placed on the surface of the agar medium and a replica of the colonies was obtained.

3) The replica filter was transferred to a selective agar medium containing chloramphenicol with the colonies upside, and incubated overnight at 37 °C. On the other hand, the master plate was incubated at 37 °C for 2 to 3 hours, and stored at 4 °C.

4) The replica filter was transferred onto filter paper wetted with 0.5M NaOH/1.5M NaCl and left to stand for 10 to 15 minutes at room temperature.

5) To neutralize the filter, it was transferred onto filter paper wetted with 1M Tris-HCl (pH 7.0)/1.5M NaCl, and left to stand for 2 to 3 minutes.

6) The filter was lightly rinsed with 3 x SSC for 15 to 20 seconds, and dried over dry filter paper.

7) The filter was baked under vacuum at 80 °C for 2 hours.

8) To remove the cell debris, the filter was lightly shaken at 65 °C with 3 x SSC for 1 hour. This washing was repeated twice, and then the surface of the filter was lightly rubbed with fingers. The washing was repeated twice further.

9) The filter was dried in the air on dry filter paper.

10) The synthetic nucleotide obtained in Example 2 was labelled at its ends with $^{32}$P to prepare a probe. The specific activity of the probe was 7.8 x 10$^7$ to 4.4 x 10$^8$ cpm/microgram of DNA.

11) The hybridization conditions were as follows.

Hybridization solution

| 50 x Denhardt | 0.2 ml |
|---|---|
| 30 X NET | 0.4 ml |
| 20 % dextran sulfate | 1.0 ml |
| 10 % SDS | 0.1 ml |
| H$_2$O | 0.3 ml |
| $^{32}$P-labelled probe Final concentration | 5 x 10$^5$ cpm/ml |
| (The above values are the amounts for each nitrocellulose filter.) | |

Hybridization temperature (°C)

T = {(A + T)x2 + (G + C)x4} - 4

where

T:      hybridization temperature (°C)
A:      adenine
T:      thymine
G:      guanine
C:      cytosine
(Note): When T is at least 55 °C, the hybridization is carried out at 50 °C.
Hybridization time:
16 to 18 hours.
12) After the hybridization, the filter was washed at a suitable temperature ranging from room temperature to 37 °C in 6 x SSC to remove the probe.
13) The filter was then air-dried on dry filter paper.
14) The filter was autoradiographed.
15) The positive colonies which hybridized with the radioactive probe were collected as dark spots.
By the above method, transformants containing the plasmid vector which underwent site-specific mutation were obtained.

EXAMPLE 5

DNA base sequence of a vector containing improved colicin E1 promoter

The colonies selected in Example 4 were cultivated in an LB medium containing 12.5 microgams/ml of tetracycline. The resulting cells were treated by the method of Birmboim et al. [Nucleic Acids Res., 7, 1513 (1979)] to obtain a plasmid. The plasmid was digested with FnuDII and TaqI, and the base sequence of the resulting fragment containing colicin E1 promoter region was determined by the dideoxy method [Sarger et al.: Science, 214, 1205 (1981), Messing, Methods in Enzymology, 101, 20 (1983)]. This led to the determination that the colicin E1 promoter region was mutated as shown below. The plasmid vectors were named pHT35-1, pHT10-6 and pHT35-2, respctively.

(a) Vector: pHT35-1

**(before improvement)**

5'-CAGTCTTGACAGGGAAAATGCAGCGGCGTAGCTTTTA
TGCT-3'

**(after improvement: pHT35-1)**

5'-CAGTCTTGACATGGAAAATGCAGCGGCGTATAATTTA
TGCT-3'

(b) Vector: pHT10-6

**(before improvement)**

5'-CAGTCTTGACAGGGAAAATGCAGCGGCGTAGCTTTTA
TGCT-3'

**(after improvement: pHT10-6)**

5'-CAGTCTTGACAGGGAAAATGCAGCGGCGTATAATTTA
TGCT-3'

EXAMPLE 6

Experiment on Expression

Microorganism strains bearing the two plasmid vectors obtained in Example 5 were subjected to induction by treatment with mytomycins. Using the ability to produce human Cu.Zn-SOD as an index, the strength of improved colicin E1 promoter was measured.

Specifically, the microorganism strains bearing the plasmid vectors were obtained by transforming E. coli 545πHR strain with the plasmid vectors by a known method, and selected as tetracycline-resistant microorganism strains. pUBE2-bearing E. coli was used as a control and subjected to induction.

Each of the plasmid-bearing E. coli strains was inoculated in 20 ml of M9 medium to which 0.3 % of Casamino acid (a product of Difco Company), 0.4 % of glucose and 12.5 micrograms/ml of tetracycline had been added; and cultivated with shaking at 37 °C. When the $OD_{660}$ of the culture reached about 0.4, 2 ml of 10-fold concentrated M9 buffer, 0.2 ml of 40 % glucose and 0.1 ml of mitomycin C (0.4 mg/ml) were added. The mixture was left to stand at 37 °C for 2 hours to perform induction.

In order to measure the strength of the improved colicin E1 promoter in terms of the ability to produce human Cu.Zn-SOD protein, 1.5 ml of the culture broth was taken into an Eppendorf tube. The cells were harvested by centrifugation at 15,000 rpm for 30 seconds, washed with 1 ml of a 50 mM phosphate buffer (pH 7.0) and then resuspended in 100 microliters of phosphate buffer. Forty microliters of the suspension was separated and an equal volume of a lyzate solution [4 % SDS, 20 % glycerol, 1.4M beta-mercaptoethanol, 20 mM phosphate buffer (pH 7.0), 0.01 % BPB] was added, and the mixture was boiled at 100 °C for 3 minutes to prepare a sample for protein electrophoresis.

The protein electrophoresis was carried out by a known Laemmli method [Laemmli, Nature <u>227</u>, 830-685 (1970)] using a concentrating gel (3 %) and a separating gel (12.5 %).

The amount of human Cu.Zn-SOD protein produced in a soluble protein fraction was measured by a densitometer (DENSITRON MICON 20, made by Jokosha). The results are shown in Table 1.

9

Table 1

| Plasmid vector | Amount (%) of human Cu.Zn-SOD protein |
|---|---|
| pUBE2 | 5.2 |
| pHT35-1 | 25.0 |
| pHT10-6 | 17.6 |

EXAMPLE 7

Experiment on expression:-

The strength of the promoter in plasmid vector pHT35-1-bearing microorganism (FERM P-9435) and pUBE2-bearing microorganism were compared by using a 2.6-liter jar fermentor (made by Marubishi Bio-Engineering Co., Ltd.). Specifically, 50 ml of a pre-culture of each of the microorganisms was inoculated in 1 liter of M9 medium to which 0.3 % of Casamino acid, 0.4 % of glucose and 12.5 micrograms/ml of tetracyclines had been added. The inoculated microorganism was cultivated at 37 °C. When the $OD_{660}$ reached about 0.4, mitomycin C was added so that its final concentration became 2 micrograms/ml. The cultivation was continuously carried out to induce synthesis. A portion (1.5 ml) of the culture broth was sampled, and the strength of the promoter was measured using the amount of human Cu.Zn-SOD produced in a soluble protein fraction as a promoter. The results are shown in Table 2.

Table 2

| Plasmid vector | Amount (%) of human Cu.Zn-SOD protein |
|---|---|
| pUBE2 | 9.3 |
| pHT35-1 | 28.9 |

EXAMPLE 8

Experiment on expression:-

The strength of the promoter in plasmid vector pHT35-1-bearing microorganism (FERM P-9435) and pUBE2-bearing microorganism were compared by using a 2.6-liter jar fermentor (made by Marubishi Bio-Engineering Co., Ltd.). Specifically, 50 ml of a pre-culture of each of the microorganisms was inoculated in 1 liter of M9 medium to which 0.3 % of Casamino acid, 0.4 % of glucose and 12.5 micrograms/ml of tetracyclines had been added. The inoculated microorganism was cultivated at 37 °C. When the $OD_{660}$ reached about 0.4, ultraviolet light (254 nm, 7800 $\mu$W.sec/cm$^2$) was irradiated, and the cultivation was continuously carried out to induce synthesis. A portion (1.5 ml) of the culture broth was sampled, and the strength of the promoter was measured using the amount of human Cu.Zn-SOD produced in a soluble protein fraction as a promoter. The results are shown in Table 3.

Table 3

| Plasmid vector | Amount (%) of human Cu.Zn-SOD protein |
|---|---|
| pUBE2 | 5.3 |
| pHT35-1 | 17.9 |

EXAMPLE 9

Plasmid vector having a multicloning site (see Figure 1)

A multicloning site was formed by a site mutation method in improved colicin E1 promoter having high expression ability so that a desired structural gene would be expressed.

Plasmid vector pHT35-1 was digested with DraI, and the cleavage site was dephosphorylated by bacterial alkaline phosphatase. pUC13 (a product of Pharmacia Co.) was digested with two restriction endonucleases EcoRI and HindIII to isolate a 45 bp DNA fragment containing a multicloning site (MCS). The resulting DNA chain containing MCS was divided into a single-stranded portion and a double-stranded portion by DNA polymerase (Klenow fragment). The DraI DNA fragment of pHT35-1 was bonded to the double-stranded DNA containing MCS, and the resulting recombinant DNA fragment was transducted into E. coli to prepare a plasmid vector having MCS downstream of the improved colicin E1 promoter. The MCS of the resulting plasmid vector has sites of cleavage with restriction endonucleases SstI, SmaI, XmaI, BamHI, XbaI, SalI, AccI, HincII and PstI.

E. coli 545πHR strain transformed with plasmid vector pHT 35-1 has been deposited with the Fermentation Research Institute (FRI), Japan under accession number FERM P-9435 (FERM BP-1954).

**Claims**

1.  A promoter of E. coli, in which
    (a) the -35 region in the promoter shows the following base sequence

$$\textbf{5}'\textbf{-TTGACAX-3}'$$
$$\textbf{AACTGTY}$$

    wherein the XY base pair represents a base pair of either TA, GC, AT or CG,
    and
    the -10 region in the promoter shows the following base sequence

$$\textbf{5}'\textbf{-TATAAT-3}'$$
$$\textbf{ATATTA}$$

    and,
    (b) an intermediate region between the -35 region and the -10 region in the promoter consists of 15 to 17 base pairs of which at least 60% are base pairs CG and GC.

2.  A promoter according to claim 1 in which the XY base pair is a base pair TA or GC.

3.  A promoter according to claim 1 or 2 in which the -35 region is composed of base pairs from the -30 base pair to the -36 base pair.

4.  A promoter according to claim 1, 2 or 3 in which the -10 region is composed of base pairs from the -8 base pair to the -13 base pair.

5.  A promoter of claim 1 in which the base sequences of the -35 region, the intermediate region and the -10 region in the promoter are selected from the following two base sequences:

$$\textbf{5}'\textbf{-TTGACATGGAAAATGCAGCGGCGTATAAT-3}'$$
$$\textbf{AACTGTACCTTTTACGTCGCCGCATATTA} \quad , \quad \text{and}$$

```
5'-TTGACAGGGAAAATGCAGCGGCGTATAAT-3'
AACTGTCCCTTTTACGTCGCCGCATATTA
```

**6.** A promoter according to any one of the preceding claims which is obtainable by replacing the bases of colicin E1 promoter.

**7.** A vector comprising a promoter of E. coli as claimed in any one of claims 1 to 7.

**8.** A vector according to claim 7 comprising also a base sequence selected from
(a) a base sequence having a plurality of sites of cleavage with restriction endonucleases and
(b) a base sequence showing a structural gene of a protein
downstream of the promoter.

**9.** A vector according to claim 8 in which the structural gene of a protein is a gene of superoxide dismutase having biological activity.

**10.** A vector according to claim 7, 8 or 9 which is a plasmid.

**11.** Escherichia coli K12 545$\pi$HR (pHT35-1) (FERM P-9435) (FERM BP-1954).

**Patentansprüche**

**1.** Ein Promotor von E. coli, bei dem
(a) die -35 Region im Promotor die folgende Basensequenz

```
5'-TTGACAX-3'

    AACTGTY
```

aufweist, worin das XY Basenpaar eines der Basenpaare TA, GC, AT oder CG bedeutet, und die -10 Region im Promotor die folgende Basensequenz

```
5'-TATAAT-3'

    ATATTA
```

aufweist, und
(b) eine Zwischenregion zwischen der -35 Region und der -10 Region im Promotor aus 15 bis 17 Basenpaaren besteht, von denen mindestens 60% Basenpaare CG und GC sind.

**2.** Promotor nach Anspruch 1, bei dem das XY Basenpaar ein Basenpaar TA oder GC ist.

**3.** Promotor nach Anspruch 1 oder 2, bei dem die -35 Region aus Basenpaaren vom -30 Basenpaar bis zum -36 Basenpaar besteht.

**4.** Promotor nach Anspruch 1, 2 oder 3, bei dem die -10 Region aus Basenpaaren vom -8 Basenpaar bis zum -13 Basenpaar besteht.

**5.** Promotor nach Anspruch 1, bei dem die Basensequenzen der -35 Region, der Zwischenregion und der -10 Region im Promotor aus den folgenden beiden Basensequenzen

```
5'-TTGACATGGAAAATGCAGCGGCGTATAAT-3'
   AACTGTACCTTTTACGTCGCCGCATATTA
```

und

```
5'-TTGACAGGGAAAATGCAGCGGCGTATAAT-3'
   AACTGTCCCTTTTACGTCGCCGCATATTA
```

ausgewählt sind.

**6.** Promotor nach einem der vorangehenden Ansprüche, der erhältlich ist, indem die Basen von Colicin E1-Promotor ersetzt werden.

**7.** Ein Vektor, der einen Promotor von E. coli gemäß einem der Ansprüche 1 bis 7 enthält.

**8.** Vektor nach Anspruch 7, der ebenfalls eine Basensequenz, ausgewählt aus
  (a) einer Basensequenz mit mehreren Stellen der Spaltung mit Restriktions-Endonucleasen und
  (b) einer Basensequenz, die ein Strukturgen eines Proteins aufweist,
stromabwärts des Promotors enthält.

**9.** Vektor nach Anspuch 8, bei dem das Strukturgen eines Proteins ein Gen von Superoxid-Dismutase mit biologischer Aktivität ist.

**10.** Vektor nach Anspruch 7, 8 oder 9, der ein Plasmid ist.

**11.** Escherichia coli K12 545πHR (pHT35-1) (FERM P-9435) (FERM BP-1954).

**Revendications**

**1.** Promoteur d'E. coli dans lequel
  a) la région -35 du promoteur présente la séquence de bases suivante :

```
5'-TTGACAX-3'
   AACTGTY
```

dans laquelle la paire de bases XY représente une paire de bases TA, GC, AT ou CG,
et la région -10 du promoteur présente la séquence de bases suivante :

```
5'-TATAAT-3'
   ATATTA
```

et
  b) la région intermédiaire entre la région -35 et la région -10 du promoteur est constituée de 15 à 17 paires de bases dont au moins 60 % sont des paires de bases CG et GC.

**2.** Promoteur conforme à la revendication 1, dans lequel la paire de bases XY est une paire de bases TA ou GC.

**3.** Promoteur conforme à la revendication 1 ou 2, dans lequel la région -35 est constituée par les paires de bases allant de la paire de bases -30 à la paire de bases -36.

EP 0 314 274 B1

4. Promoteur conforme à la revendication 1, 2 ou 3, dans lequel la région -10 est constituée par les paires de bases allant de la paire de bases -8 à la paire de bases -13.

5. Promoteur conforme à la revendication 1, dans lequel les séquences de bases de la région -35, de la région intermédiaire et de la région -10 du promoteur sont choisies parmi les deux séquences de bases suivantes :

```
5'-TTGACATGGAAAATGCAGCGGCGTATAAT-3'
   AACTGTACCTTTTACGTCGCCGCATATTA      ,
```

et

```
5'-TTGACAGGGAAAATGCAGCGGCGTATAAT-3'
   AACTGTCCCTTTTACGTCGCCGCATATTA
```

6. Promoteur conforme à l'une quelconque des revendications précédentes, que l'on peut obtenir en remplaçant des bases du promoteur colicine E1.

7. Vecteur comprenant un promoteur d'E. coli conforme à l'une quelconque des revendications 1 à 7.

8. Vecteur conforme à la revendication 7, comprenant aussi, en aval du promoteur, une séquence de bases choisie parmi
   a) une séquence de bases comportant plusieurs sites de coupure par des endonucléases de restriction, et
   b) une séquence de bases comportant un gène structural de protéine.

9. Vecteur conforme à la revendication 8, dans lequel le gène structural de protéine est un gène de superoxyde dismutase possédant une activité biologique.

10. Vecteur conforme à la revendication 7, 8 ou 9, qui est un plasmide.

11. Escherichia coli K12 545πHR (pHT35-1) (FERM P-9435) (FERM BP-1954).

14

# FIG. 1